# EUROPEAN PATENT SPECIFICATION

(11) **EP 1 204 761 B1**
(45) Date of publication and mention of the grant of the patent: **02.04.2008**
(21) Application number: 00957535.8
(22) Date of filing: 18.08.2000
(51) Int. Cl.: C12N 15/87, C12N 15/86, A61K 47/48, A61K 48/00

(54) **TARGETED ARTIFICIAL GENE DELIVERY**
GEZIELTE VERABREICHUNG VON KUENSTLICHEN GENEN
TRANSFERT CIBLES DE GENES ARTIFICIELS

(30) Priority: 19.08.1999 US 377153
(43) Date of publication of application: 15.05.2002
(73) Proprietor: UNIVERSITY OF SOUTHERN CALIFORNIA, Los Angeles, CA 90007 (US)
(72) Inventor: ROZENBERG, Yanina, Studio City, CA 91604 (US); MEDVEDKIN, Viacheslav, Los Angeles, CA 90004 (US); ANDERSON, W., French, San Marino, CA 91108 (US)
(74) Representative: LOUIS, PÖHLAU, LOHRENTZ
(86) International application number: PCT/US2000/022619
(87) International publication number: WO 2001/012235

(56) References cited:
- WO-A-00/02909
- WO-A-96/21036
- WO-A-97/40854
- WO-A-98/44938
- US-A- 4 948 590
- US-A- 5 631 018
- FASBENDER A ET AL: "COMPLEXES OF ADENOVIRUS WITH POLYCATIONIC POLYMERS AND CATIONIC LIPIDS INCREASE THE EFFICIENCY OF GENE TRANSFER IN VITRO AND IN VIVO" JOURNAL OF BIOLOGICAL CHEMISTRY,AMERICAN SOCIETY OF BIOLOGICAL CHEMISTS, BALTIMORE, MD,US, vol. 272, no. 10, 7 March 1997 (1997-03-07), pages 6479-6489, XP002060642 ISSN: 0021-9258 cited in the application
- JANUSZESKI M M ET AL: "Functional analysis of the cytoplasmic tail of Moloney murine leukemia virus envelope protein" JOURNAL OF VIROLOGY,US,THE AMERICAN SOCIETY FOR MICROBIOLOGY, vol. 71, no. 5, May 1997 (1997-05), pages 3613-3619, XP002130560 ISSN: 0022-538X

### SEQUENCE LISTING

<110> ROZENBERG, Yanina
   MEDVEDKIN, Viacheslav
   ANDERSON, W. French
<120> TARGETED ARTIFICIAL GENE DELIVERY
<130> 023465/0107
<140> US 09/377,153
   <141> 1999-08-19
<160> 1
<170> PatentIn Ver. 2.0
<210> 1
   <211> 19
   <212> PRT
   <213> Artificial Sequence
<220>
   <223> Description of Artificial Sequence: Targeting ligand peptide
<400> 1
   Ile Leu Asn Arg Leu Val Gln Phe Val Lys Asp Arg Ile Ser Val Val
     1               5                  10                  15
   Gln Ala Leu

## Description

### BACKGROUND OF THE INVENTION

### 1. Field of the Invention

The invention provides improved vectors for cell-specific gene delivery to a target cell. The vectors according to the instant invention comprise a recombinant core containing the genetic materials to be delivered and an artificially reconstituted surface encompassing the core. The surface facilitates targeting and cell fusion of the vector, and also provides an immunoprotection function for the vector. Methods for preparing the vectors and for transfecting eukaryotic cells using the vectors also are disclosed.

### 2. Description of the Related Art

Gene therapy has received a great deal of attention for its potential of providing effective treatment of many human diseases, ranging from rare heritable genetic defects and common diseases such as cancer, AIDS, hypertension, atheroma and diabetes. The great potential of gene therapy has up until now been hampered by the lack of efficient vector systems for delivery of genetic constructs into cells *in vivo* and *ex vivo.*

As with the targeted delivery of conventional drugs, one of the goals of genetic therapy is to maximize the local therapeutic effect of gene delivery while minimizing the potential for systemic adverse events. To achieve this goal, an ideal gene delivery vector should possess several attributes. First, the vector should be able to reach a target site within the organism, and preferably should be able to recognize the specific cell types. This requires that the vector have low immunogenicity as well as targeting characteristics. Second, the vector should be able to cross the membrane barrier of host cells to deliver its therapeutic genetic material into the inside of the cells. The capacity of the current vector systems is in most cases large enough to accommodate the delivery of the desired genetic constructs. Third, once within a cell, the vector must be able to unpackage its genetic load to allow efficient gene expression. The expression preferably is cell-specific, and is non-harmful overall. In most applications the vector should lack the ability to autonomously replicate its own DNA. Fourth, where necessary, the vector should provide controlled, sustained gene expression over an extended time period. Fifth, the vector should be amenable to manufacture on a commercial scale, and be available in a pharmaceutically deliverable, concentrated form.

None of the delivery systems currently available for gene therapy is satisfactory with respect to all of these attributes. Progress in gene therapy therefore depends heavily on the development of new and improved gene vector systems.

Presently, the most commonly used methods for therapeutic gene delivery *in vivo* are viral delivery systems and cationic polymer or lipid-based systems. In viral based systems, the natural cell penetration ability of the viruses is retained in the genetically modified viruses manipulated to deliver therapeutic genes. In polymer or lipid-based systems, therapeutic DNA is condensed with one or more cationic polymers and/or cationic lipids, and cellular delivery exploits the attraction between the negatively charged cell and positively charged gene delivery particle. In the majority of current applications, the targeting of specific cell types has not been achieved.

Viral vectors as a class suffer several significant failings, such as the inability to efficiently escape the host immune system, restrictions on the types of cell that can be infected, difficulties in producing vectors with high titers, limits on the ability to package large DNA or RNA molecules, and integration into the host genome, which is advantageous for stable expression, yet produces a finite, albeit low, chance of an undesirable insertion into a functional genomic site. Gene transfer using nucleic acids encapsulated into agents such as polymers or lipids has the ability to transfect a broad spectrum of host cells *in vitro*, but also suffers from problems for *in vivo* delivery such as inability to evade the immune system, lack of cell specificity, low efficiency of cell entry due to the lack of entry mechanisms, and low efficiency of vector unpackaging once within a cell.

The combination of viral and non-viral elements can be used to increase the efficiency of gene transfer to cells. For example, Fasbender et al. (J. Biol. Chem. 272:6479-6489 (1997)) described the incorporation of adenoviral lysosomal degradation escape functions into an artificially packaged DNA formulation in order to enhance the delivery efficiency of the encapsulated DNA. Another example of a combination of viral and non-viral elements uses plasmids containing the inverted terminal repeat (ITR) sequences of adeno associated virus (AAV) complexed to cationic liposomes, where gene transfer and subsequent interleukin-2 (IL-2) gene expression was 3-10 times higher than the levels obtained with plasmids lacking the ITRs. Vieweg et al,. Cancer Research 55: 2366-2372(1995)). In another example, adenoviral capsid proteins or adenoviral fiber proteins were combined with liposomes, providing an increased transfection efficiency of a reporter gene. Hong et al., Chinese Medical J. 108:332-337 (1995).

Despite these recent developments, none of the currently available methods for targeted gene delivery is satisfactory in simultaneously providing low immunogenicity, increased vector stability, sufficient targeting versatility, and increased gene expression efficiency. It is, therefore, a goal of the present invention to overcome these difficulties in the art and to provide a versatile vector for targeted gene delivery and expression.

### SUMMARY OF THE INVENTION

Accordingly, it is an object of the present invention to provide an improved, non-naturally occurring, gene therapy vector for cell-specific delivery of nucleic acid to a target cell.

In accomplishing these and other objects, there has been provided, according to one aspect of the present invention, a non-naturally occurring gene therapy vector according to claim 1 comprising a recombinant core and a non-naturally occurring functional surface moiety, where the said core comprises a nucleic acid molecule, and where at least one expression product of the vector is a therapeutic nucleic acid, peptide or protein, where the functional surface moiety comprises an immuno-protective element, a targeting element, and a cell-entry element, and where the vehicle is capable of specifically binding to and delivering the core into a target cell.

According to one embodiment of the invention, the core further comprises at least one viral capsid protein. The functional surface moiety comprises an immunoprotective element, a targeting element, and a cell-entry element.

The immunoprotective element comprises a synthetic polymer moiety. The synthetic polymer component may comprise a poly(ethyleneglycol). The synthetic polymer component comprises a copolymer of glutamic acid with leucine.

In accordance with another aspect of the invention, the targeting moiety binds to a receptor that is more highly expressed in diseased cells than in normal cells. The targeting moiety is a peptide ligand for a cell surface receptor.

The cell-entry element is a membrane-destabilizing moiety comprising an amphiphilic α-helix. The amphiphilic α-helix may be derived from the C-terminal domain of a viral *env* protein. In a particular embodiment, that C-terminal domain is the C-terminal domain of the Moloney leukemia virus env protein. That C-terminal domain may comprise amino acids 598-616 of the Moloney leukemia virus env protein. In another embodiment, the membrane-destabilizing moiety comprises a copolymer of glutamic acid with leucine.

Other objects, features and advantages of the present invention will become apparent from the following detailed description. It should be understood, however, that the detailed description and the specific examples, while indicating preferred embodiments of the invention, are given by way of illustration only.

### BRIEF DESCRIPTION OF THE DRAWINGS

Figure 1 shows a schematic diagram of a TAGD particle surface containing an immunoprotective element (PEG), a fusogenic element (the membrane destabilizing peptide) and a cell binding element (the targeting peptide).
Figure 2 shows two different methods by which ligands can be incorporated into the surface to generate a TAGD particle.
Figure 3 shows DSPE-PEG-rhodamine (red fluorescence) associated with viral particles after incubation of the virions with micelles containing DSPE-PEG-rhodamine.
Figure 4 shows that chemically modified MoMuLV containing a polyfunctional and polyvalent linker containing an α-MSH peptidomimetic ligands was able to bind human melanoma D10 cells, whereas unmodified virus did not bind.

### DETAILED DESCRIPTION OF THE PREFERRED EMBODIMENTS

The present invention provides novel improved compositions and methods for gene therapy. In particular, a targeted artificial gene delivery ("TAGD") vehicle is provided, comprising a multifunctional artificial surface moiety surrounding a recombinant viral particle (nucleocapsid) or recombinant core.

The functional surface contains molecular elements that enable the vector to evade the host's immune system, to recognize and bind to specific target cells, and to efficiently fuse with the target cell and deliver a transgene encoded by the core gene complex into the target. The surface molecular elements can contain, for example, multiple peptides and immunoprotective elements.

In one embodiment, the invention provides vectors where an existing lipid envelope moiety of a recombinant virus is modified to enhance or provide the desired characteristics of the viral particle. For example, a viral surface can be manipulated to add immunoprotective elements, for example polymer groups such as poly(ethyleneglycol) (PEG), which decrease the immunogenicity of the viral particle, and also increase the stability of the vector in blood circulation. The surface also can be modified by addition of targeting ligands that enhance the cell specificity of the virus. For example, using methods described in more detail below, the surface can be engineered to include a ligand molecule that can specifically interact with a receptor of choice on the surface of a target cell.

In another example, the surface is modified to include elements that enhance the cell entry properties of the vector. In particular, fusogenic peptides, proteins, or polymers may be used to enhance entry of the vector into the target cell. A fusogenic moiety promotes fusion of the vector to the cell membrane of the target cell, facilitating entry of the viral core into the cell cytoplasm. The fusogenic moiety may be a naturally occurring fusogen, such as a viral fusogenic peptide, or may be an engineered (non-naturally occurring) moiety, such as a peptide containing a membrane-active amphiphilic α-helix or some other conformational feature that provides membrane destabilization. Alternatively, the fusogenic moiety can be a polymer that provides membrane destabilization.

In each of these embodiments, modifications to the existing surface of a gene delivery particle can be made non-covalently, where lipids modified by chemical conjugation to contain functional moieties are incorporated into the surface of the core containing particle. This incorporation is done, for example, by forming micelles containing the desired modifying moiety, where the micelles are spontaneously fused to the **surface of a particle using methods that are known in the art. Non-covalent modifications** also can be made by adsorbing functional components onto the the surface of a particle by electrostatic and/or hydrophobic (van der Waals) interactions.

Alternatively, the modifications can be made by covalent modification of the constituents of the surface of a gene delivery particle. Thus, for example, in the case of enveloped viruses, the membrane lipids on the viral surface can be linked to chemically activated linkers to allow subsequent covalent attachment of modifying groups. The carbohydrate groups on membrane glycoproteins can be chemically oxidized using methods that are well known in the art to produce reactive aldehyde groups, which permit covalent modification with, for example, polymeric materials or peptides containing free amino groups. Other methods of covalent modification of membrane constituents are well known in the art.

In another example, an existing viral surface can be chemically modified at the lipid and/or protein components by reaction with activated compounds. Activated linker compounds include, but are not limited to, the incorporation of highly reactive chemical groups, for example: sulfonyl halides and/or various active esters of carboxylic acids for the modification of amino groups; hydrazides for modification of carbohydrates; maleimidoyl or reactive alkylhalides for modification of thiol groups; and photoactivated groups that are known in the art. The skilled artisan will be aware that the use of other chemically activated groups is also possible.

As another alternative, functional groups on the surface of a gene delivery particle can be chemically modified using methods known in the art to produce reactive and/or chemically selective groups, which permit covalent modification with, for example, polymeric materials or peptides containing free amino groups. A specific example is modification of the viral surface with 2-iminothiolane (Traut's reagent), which converts accessible amino groups on the viral surface into sulfhydryl groups. This allows for enhanced selectivity of modification by using thiol-selective methods of conjugation. Modification of the viral surface with Traut's reagent also provides a very convenient means for estimating the optimal effective level of the chemical modification of various viruses. If the level of chemical modification is too low, the properties of the viral surface will not be changed as desired, whereas too high a level of modification will inactivate the virus. The present inventors have found that even very unstable Moloney Leukemia virus allows appreciably high loading of chemical substances on its surface without a functionally significant loss of viral titer. Methods of measuring viral titer following chemical modification are known in the art.

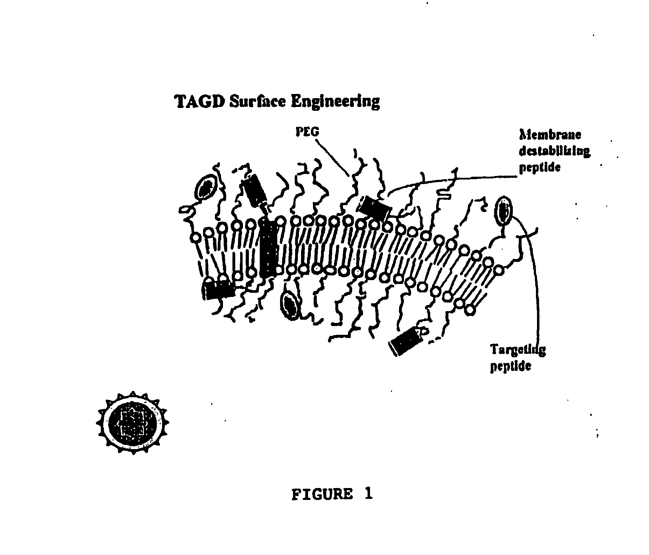

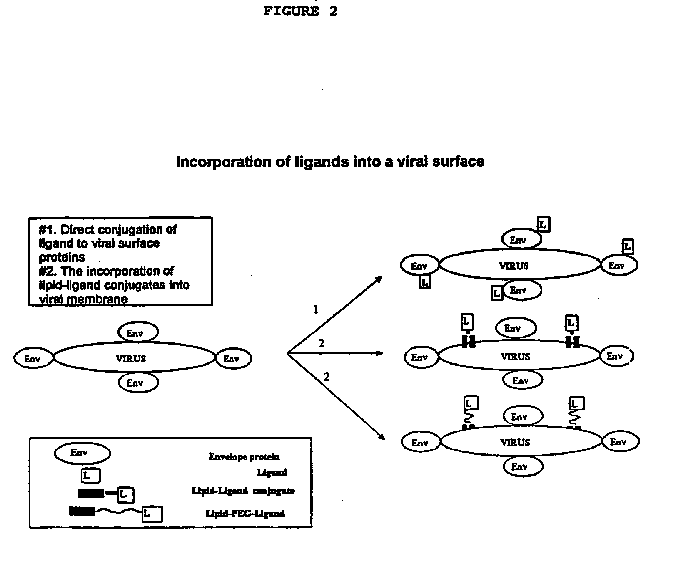

In another embodiment the functional group that is to be introduced into the vector, such as PEG or targeting or fusogenic peptides, is first conjugated to an activated linker. This linker preferably possesses hydrophobic loci for better affinity to the surface of the gene delivery particle. For example, a copolymer of glutamic acid and leucine (described in more detail below) maybe used. The linker also may carry a positive charge for better affinity to the cell surface. Some proportion of the active groups can be retained (or created *de novo)* for the covalent attachment of the linker to the surface of a particle. For example, this linker can be used to modify the surface of viruses in multiple ways without exposing fragile viruses to excessive chemical treatment. Figure 1 shows a schematic diagram for a surface of TAGD particle containing an immunoprotective element (PEG), a fusogenic element (the membrane destabilizing peptide) and a cell binding element (the targeting peptide). Figure 2 shows two different methods by which ligands can be incorporated into the viral surface.

In a further embodiment, vectors are provided where the vector contains a recombinant core within a surface that is prepared *de novo.* This embodiment is suitable for use with viral core particles whether or not the particle contains an existing membrane. Thus, for example, a recombinant viral particle that lacks an outer membrane layer can be encased within an artificially generated lipid membrane. A recombinant viral particle that contains an outer membrane layer can be modified (i) by chemical methods, (ii) by encapsulation within a new membrane, or (iii) by replacement of the existing membrane, to contain new surface elements synthesized *de novo.* Thus, the surface of the resulting particles can be engineered to contain immunoprotective, fusogenic, and cell-specificity enhancing agents as described above. In addition, the membrane can contain components that enhance the stability of the vector in the circulation of the host, thereby improving the chance that the vector will reach its intended target, and also providing an opportunity for increased duration of action of the vector.

### The viral core

The viral core moiety of the vector may be any recombinant viral core that is suitable for use in gene therapy. A wide variety of suitable viral cores can be prepared by methods that are well known in the art and can be used as the internal components of the TAGD vehicles. For examples of viruses used in gene therapy *see* Jain, "Textbook of Gene Therapy", Chapter 4, pp. 35-66 (Hogrefe &Huber, 1998). Suitable viral cores include recombinant viruses, for example, replication-defective lentivirus, such as HIV, or other retroviruses, for example, murine leukemia viruses (MLV) such as Moloney murine leukemia virus (MoMLV) or Friend MLV, adenovirus, adeno-associated virus (AAV), herpes simplex type 1 (HSV-1), vaccinia virus, Epstein-Barr virus, rabies and pseudorabies virus, Sindbis virus, SV40, and cytomegalovirus, influenza virus, baculovirus, Semliki Forest Virus(SFV), and vesicular Stomatitis virus (VSV), *etc.* These viruses may be engineered so as to lack a functional env gene if necessary. Removal of the *env* gene by recombinant methods reduces the immunogenicity of the virus, and also may increase the production levels of virions in cell culture. Viruses in which the env gene has been removed lack a natural ability to bind and fuse to target cells. Accordingly, these abilities must be provided by the TAGD vector.

Methods for manipulating each of these viral cores to contain an exogenous gene suitable for use in gene therapy, and for removing or substituting progressively larger segments of native viral genomes for enhanced safety, are well known in the art. See for example. Jain, *supra,* Anderson, Nature, 392 (6679 Suppl):25-30 (1998): Verma et al. Nature, 389:239-42 (1997); WO 91/19798; WO 97/12622; WO 98/12314; and US Patent Nos. 5,665,577 and 5,686,279. See also, generally, Fields et al., "Virology" (Lippincott-Raven, 1996).

The skilled artisan will recognize that the methods and compositions described herein are also suitable for use with viral core analog (nucleic acid) cores that may be developed in the future.

### Immunoprotective elements

In the context of the invention, an immunoprotective element is a molecule or collection of molecules that are disposed on the surface of the vectors and thereby reduce the immunogenicity of the vector. That is, when the vector is administered to a patient, the element reduces the interaction of the vector with the components of the patient's serum, thus avoiding activation of the immune system, and lowering the *in vivo* clearance rate of the vector in the patient.

Examples of immunoprotective strategies include decreasing the charge of a drug delivery particle and/or its molecular mass, and decreasing the particle's hydrophilicity by glycosylation. The most widely used approach is to attach "immuno-decoy" elements to the surface of a particle that reduce recognition of the particles by the opsonins in blood serum, thereby delaying or avoiding entirely activation of the immune system. One method of attaching decoy polymers to a drug delivery vehicle is *via* polymer conjugation to lipids. The polymer most commonly for this purpose used is poly(ethylene glycol) (PEG) (Papahadjopoulos et al., Proc Natl Acad Sci USA. 15:11460-4 (1991). Other elements used for the same purpose are: sialic acid GM1 polyglycerols (Schauer, TIBS Sept. 1985:357, Yamanauchi et al., J. Controlled Release 113:141(1995): polyoxazoline-DSPE (distearoylphosphatidylethanolamine) derivatives (Zalipsky et al., J Pharm Sci. 85:133-7 (1996)), polymer-based 'stealth' approaches (Torchilin et al., J Pharm Sci. 84:1049 (1995)), phosphatidylpolyglycerol (Maruyama et al., Biochim Biophys Acta. 1234:74-80 (1995)), fleximer polymers ((Papisov. Adv. Drug Delivery Review 32, 119-138 (1998); Torchilin J. Microencapsulation 15, 1-19 (1998)). Alternative immunoprotective elements include, but are not limited to, polyglutamic acid, polylactic acid, polyglycolic acid, polyvinylpyrrolidone, polymethacrylamide, polyethyloxazoline, polymethyloxazoline, and polyvinylalcohol. As described above for PEG, each of these hydrophilic polymers can, if necessary, be covalently coupled to the vector through a reactive chemical group. The present invention also provides a novel combinatorial linker based on copolymers of glutamic acid with leucine that can be used to introduce desired function to the surface of a particle and that has immunoprotective function. The experienced artisan in the field will recognize that glutamic acid contains a hydrophilic carboxyl side chain, where as leucine contains a hydrophobic side chain. Each of these components can be substituted with other components that share functional characteristics. Accordingly, glutamic acid can be substituted by, for example, aspartic acid, and leucine can be substituted by, for example, alanine, phenylalanine, isoleucine, valine and other hydrophobic amino acids. Natural or non-natural amino acids may be used. As an alternative, the hydrophobic function can be introduced as an ester or amide derivative of glutamic acid. Notably, random copolymers and/or block copolymers may be used for the analogous purposes.

When PEG is used as the immunoprotective element, the PEG preferably has a molecular weight of between about 1,000 to about 5,000 daltons. Advantageously, PEG having a molecular weight of about 2000 daltons is used to achieve the immunoprotection. To position a PEG chain containing a reactive functional group on the surface of the membrane-containing drug delivery vector PEG may be conjugated to a phospholipid such as desteroyl phosphatidylethenolamine (DSPE). The skilled artisan will be aware that the use of other lipids is possible. Suitable DSPE-PEG molecules that contain further modifiable PEG are well known in the art and also can be purchased from, for example, Shearwater Polymers (Huntsville, AL).

Incorporation of an immunoprotective element into a surface of a gene delivery vector, such as a viral core surrounded by a membrane, can be carried out via micelle formation. See, for example, Uster et al., Febs Lett. 386:243 (1996). Molecules of DSPE-PEG are amphiphilic and spontaneously form micelles that are thermodynamically unstable and fuse with larger membrane surfaces of the virions. Successful insertion in this manner can be verified using the methods described below. The effect of incorporation of immunoprotective elements on the blood clearance rate of particles also can be monitored using isotope-labeled particles.

For targeted gene delivery a multivalent and multifunctional linker presents many advantages. The present invention provides a novel multivalent and multifunctional linker, based on activated copolymers of acidic and hydrophobic amino acids, like glutamic acid and leucine, and functional equivalents thereof, as detailed below. Activation of such a linker can be performed by reacting the polymer with di-pentafluorophenylcarbonate in the presence of a tertiary amine. The skilled artisan will be aware that the use of other alternative means of activation such as N, N-dicyclohexylcarbodiimide-hydroxysuccenemide, BOP-reagent, and the use of other condensing reagents known in peptide chemistry is possible. Desired functional groups such as PEG or targeting or fusogenic peptides may then be conjugated to the activated linker. This linker possesses hydrophobic loci for better affinity to the viral membrane, and also retains some positive charges for better affinity to the cell surface. Some proportion of the active groups can be retained (or created *de novo)* for the covalent attachment of the linker to the carrier. This linker may be used to modify the surface of viruses in multiple ways without exposing fragile viruses to excessive chemical treatment.

### Targeting elements

A targeting element potentiates highly specific attachment of the vector to the target cell membrane. For example, a targeting element may be a targeting polypeptide containing a binding region that binds to a receptor or ligand on the surface of the target cell. A targeting element may be selected from the group consisting of an antibody or a fragment thereof, a receptor ligand, a complete protein, a peptide, a receptor, a non-peptidic organic molecule that serves as a ligand, a vitamin, and an inorganic co-factor for a cell-surface protein. Advantageously, the targeting element may bind to a receptor that is more highly expressed in the desired host cells targeted for genetic therapy, for example, the cancer cells.

Suitable ligands include, but are not limited to, vascular endothelial cell growth factor, for targeting endothelial cells, FGF2, for targeting blood vessels, and laminin and RGD peptides, for targeting integrin expressing cells. Other examples include (i) folate, where the composition is intended for treating tumor cells having cell-surface folate receptors, (ii) pyridoxyl, where the composition is intended for treating virus-infected CD4⁺ lymphocytes, or (iii) sialyl-Lewis^{ox}, where the composition is intended for treating a region of inflammation. The targeting moiety can be a peptidomimetic. See, for example, Kieber-Emmons et al., Current Opinion in Biotechnology 8, 435-441*;* Haubner et al. Angew. Chem. Int. Ed. Engl 36, 1374-1389 (1997); Pauletti et al., Adv. Drug Delivery Review. 27, 235-256 (1997); Boxus et al., Bioorganic and Medicinal Chemistry. 6,1577-1595(1998); Schoepfer, Bioorg. Med. 8, 2865-2870 (1998).

The peptidomimetic can be directed at a receptor or other target on cancer cells. For example, a peptidomimetic analog of α-Melanostatin, ([Nle4, D-Phe7]-α-MSH) can be used for treatment of melanoma, where melanoma cells overexpress the -α-MSH receptor. Other cancer-related peptidomimetics are known in the art. See, for example Kieber-Emmons *et al,* 1997 *supra;* Haubner *et al, supra.* Neurotensin, and the receptor-binding site of plasminogen activator also have been described as cancer-related ligands (Schnierle and Groner, Gene Ther. 3:1069-73 (1996)). Other targeting ligands are described in US Patent No. 5,916,803.

In another embodiment of the invention, suitable peptide ligands can be selected using phage display methods that are well known in the art. Suitable methods are described, for example, in US Patent No. 5,780,221. Briefly, libraries of short nucleic acid sequences encoding peptides of random sequence and pre-selected length are fused in-frame to genes encoding surface proteins of filamentous phage, and the resulting peptides are expressed (displayed) on the surface of the phage. The phage are then screened for the ability to bind, under appropriate conditions, to a target molecule, such as a cell surface receptor, immobilized on a solid support. Large libraries of phage can be used, allowing simultaneous screening of the binding properties of a large number of peptide sequences. Phages that have desirable binding properties are isolated and the sequences of the nucleic acids encoding the corresponding peptide ligands are determined. These peptides can be used as ligands directly in the methods of the present invention, or can be used as templates for the design and synthesis of peptidomimetics that are used as the targeting element.

The number of targeting moieties present on the TAGD vector surface will vary, depending on factors such as the avidity of the ligand-receptor interaction, the relative abundance of the receptor on the target cell surface, and the relative abundance of the target cell. Nevertheless, it is anticipated that at least 20-100 targeting molecules must be present on the surface of each vector to provide suitable enhancement of cell targeting. The targeting moiety may be incorporated into the TAGD vector surface using a variety of methods, for example, via a thermodynamically unstable micellar intermediate, as described in more detail below. Alternatively, the targeting moiety can be added using a polylinker in the same manner as described above for addition of PEG to the vector surface. In one embodiment, the targeting moiety can be coupled to the surface by standard linker chemistry. See for example, Hermanson, Bioconjugate Techniques(Pierce Chemical Company/Academic Press, San Diego, p785 (1996)).

For example, for a surface containing a lipid-conjugated polymer, the targeting moiety can be chemically coupled to the lipid-polymer conjugate via a "donor-acceptor" type of reaction. Thus, in one example, the targeting moiety can be designed to carry a free sulfhydryl group (donor) that can react with a maleimido group (acceptor) present on the lipid-conjugated polymer. Progress of this reaction can be monitored by observing the reduction in UV absorption at 300 nm due to loss of the maleimide chromophore, and loss of free sulfhydryl, measured using Ellman's reagent. Other coupling methods that are known in the art also can be used, for example, coupling of a nucleophilic amine group (donor) with an activated carboxyl group, such as an N-hydroxysuccinimidyl ester (acceptor). The skilled artisan will further appreciate that the donor moiety may be present on the particle surface, and the acceptor on the targeting moiety, or vice versa, as necessary. Similarly, the targeting moiety also can be coupled **directly to the lipid membrane of the TAGD vehicle, to a lipid-polymer conjugate** component of the vector, or to any other suitable disposed surface moiety present on the vector.

Once conjugated in this fashion, it is desirable to ensure that the targeting moiety retains the ability to bind its intended target. This can be done using methods that are known in the art. For example, the ability of the non-conjugated targeting moiety to bind to its cognate receptor can be compared to the binding ability of the coupled moiety. This comparison can be done using the assembled TAGD vector, but is more conveniently achieved by comparing the coupled and non-coupled moieties prior to incorporation into the TAGD vector. In circumstances where the targeting moiety is coupled directly to a component or components of the assembled or partially assembled vector, the comparison may be achieved by carrying out a model reaction where the targeting moiety is coupled to the vector component or components, and the resulting model compound then is compared with the non-conjugated targeting moiety.

One example of an indirect binding assay to verify that a conjugated targeting moiety retains the ability to bind to its target is provided by consideration of melanostatin (α-MSH) compounds. Thus, α-MSH can be synthesized with a maleimido function that can be conjugated to a DSPE conjugate of PEG that contains a sulfhydryl group (SH). The α-MSH, DSPE-PEG-SH, and DSPE-PEG-α-MSH conjugates can be compared at the same nanomolar concentrations for their ability to induce melanin formation in melanoma B16 cells. It is found that the conjugated peptide has similar activity to the non-conjugated α-MSH peptide.

As described above, incorporation of the conjugates into the TAGD vector can be achieved using the ability of micelles to fuse with larger membrane surfaces. Suitable methods are described in Kirpotin et al. (FEBS Lett. 388:115-8, (1996)). See also Uster, *supra.* For example, a lipid-PEG-¹²⁵I α-MSH conjugate may be incubated with MoMuLV viral particles. The resulting virions are purified first by step sucrose gradient, followed by discontinuous gradient centrifugation. The high isotope counts are found to associate with the fractions containing the virions. In another example, a red fluorescent DSPE-PEG-rhodamine moiety can be incorporated into virions using the same types of micelles. The MoMuLV is incubated with DSPE-PEG-rhodamine and then allowed to bind to murine or human cells. The cells are next incubated with an anti-MoMuLV *env* antibody, followed by addition of a FITC-labeled secondary antibody (green fluorescence). The resulting cells may be analyzed by FACS. It is found that a rhodamine signal, here used just as a tracer, is specifically detected on the murine cells containing viral receptors and that there is no non-specific signal detected on human cells which lack receptors for binding murine viruses.

To demonstrate that incorporation of a targeting moiety into a TAGD vector can redirect the cell binding specificity of the vector, a DSPE-PEG-α-MSH conjugate can be incubated with MoMuLV viral particles as described above, and assayed for the ability to bind to D10 human melanoma cells. Modified and non-modified MoMuLV particles are allowed to bind to the D10 cells, which then are tested for the presence of the Moloney *env*-specific FITC signal, using the methods described above. It is found that MoMuLV, which is a murine virus on its own unable to bind human cells, but once pre-treated with lipid-PEG-α-MSH conjugate can bind to human D10 cells. However, no binding is detected using MoMuLV that did not contain DSPE-PEG-α-MSH on its surface. These results demonstrate that it is possible to redirect the binding characteristics of a TAGD particle using an appropriate binding moiety. One skilled in the art will recognize that this example is merely illustrative, and will further appreciate that this result is of general applicability.

### Cell-entry elements

Cell-entry elements aid in entrance of a TAGD vector into a host or target cell by providing membrane active components for the fusion between the TAGD vehicle and the target cell. Because a major limitation in efficiency in targeted drug delivery is crossing the membrane of the targeted cell, a TAGD vehicle with a functional cell-entry element is able to enter the target cell and deliver the therapeutic compound with improved efficiency.

Advantageously, the cell-entry element is a fusogenic moiety such as a peptide, that is, a peptide with membrane destabilizing abilities. The presence of a fusogenic peptide induces formation of pores in the cell membrane by disruption of the ordered packing of the membrane phospholipids. Some fusogenic peptides act to promote lipid disorder and in this way enhance the chance of merging or fusing of proximally positioned membranes of two membrane enveloped particles of various nature (e.g. cells, enveloped viruses, liposomes). Other fusogenic peptides may simultaneously attach to two membranes, causing merging of the membranes and promoting their fusion into one. Examples of fusogenic peptides include a fusion peptide from a viral envelope protein ectodomain, a membrane-destabilizing peptide of a viral envelope protein membrane-proximal domain from the cytoplasmic tails.

Other fusogenic peptides often also contain an amphiphilic-region. Examples of amphiphilic-region containing peptides include: melittin, magainins, the cytoplasmic tail of HIV1 gp41, microbial and reptilian cytotoxic peptides such as bomolitin 1, pardaxin, mastoparan, crabrolin, cecropin, entamoeba, and staphylococcal a-toxin; viral fusion peptides from (1) regions at the N terminus of the transmembrane (TM) domains of viral envelope proteins, *e.g*. HIV-1, SIV, influenza, polio, rhinovirus, and coxsackie virus; (2) regions internal to the TM ectodomain, *e.g*. semliki forest virus, sindbis virus, rota virus, rubella virus and the fusion peptide from sperm protein PH-30 (3) regions membrane-proximal to the cytoplasmic side of viral envelope proteins *e.g.* in viruses of avian leukosis (ALV), Feline immunodeficiency (FIV), Rous Sarcoma (RSV), Moloney murine leukemia virus (MoMuLV), and spleen necrosis (SNV).

In general, such fusogenic peptides have the propensity to form an amphiphilic alpha-helical structure when in the presence of a hydrophobic surface such as a membrane. An amphiphilic peptide, the artisan will understand, is a peptide in which one side is hydrophobic and the other side is hydrophilic. In one aspect, the fusogenic peptide sequence is taken from a portion of a viral envelope protein which is 5' of the membrane spanning segment of the transmembrane portion of the envelope protein.

In the native virus, the subject amphiphilic fragment of interest is located after a hydrophobic region of the membrane-spanning segment of the transmembrane portion of the envelope protein which is often about 20 amino acids in length. In general, the fusogenic peptides of the present invention can be of various lengths. In one embodiment, the peptides include an amphiphilic amino acid sequence having from about 12 to about 35 amino acid residues. The hydrophobic membrane-spanning segments often contains near their cytoplasmic end a glycine or a proline, generally associated with a turn structure. The amphiphilicity of such membrane-proximal segments may be identified by the calculations of their hydrophobic moments. The examples of such membrane-proximal amphiphilic segments is shown in Appendix 2.

In another embodiment, the fusogenic peptide comprises an amino acid sequence which is a derivative or analogue of the amino acid sequence hereinabove described. The derivative or analogue has at least one substitution of an amino acid residue of the above-mentioned amino acid sequence. In one embodiment, the fusogenic peptide is comprised of an amino acid sequence derived from the cytoplasmic portion of the envelope protein as hereinabove described, and further includes at least a portion of the transmembrane protein.

Representative examples of fusogenic peptides derived from viruses are given in Table I below. In Table I, the negative numbers refer to amino acid residues in the C-terminal region of the predicted transmembrane region of the viral envelope protein, and positive numbers refer to the number of residues from the cytoplasmic tail, beginning at the N-terminal, of the viral envelope that are in the peptide. The boundary between the transmembrane and the cytoplasmic domains is at the first hydrophilic amino acid after the stretch of about 20 hydrophobic amino acids N-terminal to the ectodomain of the viral envelope protein Thus, for example, a peptide denoted as "-2/14" means that the isolated peptide includes (in an N-terminal to C-terminal direction), as the first two amino acid residues of the N- terminal, the two C-terminal amino acids of the predicted **transmembrane portion and the last 14 amino acid residues are the 14 N-terminal amino acids of the predicted cytoplasmic tail. Table I lists the positions of the most amphiphilic** membrane-proximal segments in a number of viral envelope proteins.

Also in Table I, the following abbreviations are used: ALV-avian leukosis virus; BLV-bovine leukemia virus; EIA-equine infections anemia; FIV-feline immunodeficiency virus; HEP C-hepatits C; HIV-human immunodeficiency virus; HTLV-human T-cell leukemia virus; hRSV-human respiratory syncytial virus; infM2-influenza M2virus; INF-influenza; MMTV-Mouse Mammary Tumor Virus; MPMV-Mason Pfizer monkey virus; RSV-Rous Sarcoma Virus; PINF-parainfluenza; SNV-spleen necrosis virus; VSV-vesicular stomatitis virus; SimSrcV-HLB- simian sarcoma virus; MoMuLV-Moloney Murine Leukemia Virus.

**Table I**

| **VIRUS** | **SEGMENT** | **VIRUS** | **SEGMENT** |
|---|---|---|---|
| ALV | -2/14 | INFA1 | -2/11 |
| BLV | -2/17 | MoMuLV | -3/14 |
| EIA | 1/52 | MMTV | -6/13 |
| FIV | -6/10 | MPMV | 1/22 |
| HEP C | 1/17 | RSV | -9/8 |
| HIV 1 | -3/11 | PINF | 1/17 |
| HIV 25YR | 1/25 | SIV239 | -5/13 |
| HTLV2 | 1/12 | SNV | -2/16 |
| HRSV | 1/21 | VSV | -2/13 |
| infM2 | 1/16 | SimSrcV-HLB | -9/8 |

In a preferred embodiment, the fusogenic peptide is the membrane-proximal cytoplasmic domain of the MoMuLV envelope protein (env). This domain has structural features conserved among a variety of viruses and contains a membrane-induced α-helix. This peptide is described in copending application Serial No. 09/112,544.

In another embodiment a copolymer of glutamic acid and leucine can be exploited as a fusogenic element. These copolymers are powerful, conformational pH-dependent hydrophilic/hydrophobic carriers that can be used to deliver various substances into the cells. See WO 97/40854. The demonstrated membrane-penetrating and membrane-disturbing properties of these copolymers are, however, used for a different purpose in the context of the present invention. Rather than delivering substances into the cell this copolymer, being attached to the surface of a gene delivery particle surface, works as a pH-dependent fusogenic factor and/or as a multivalent and multifunctional carrier for other desired functions (fusogenic, immunoprotective, targeting functions *etc.).* The fusogenic properties of the pH-dependent linker, based on copolymers of glutamic acid with leucine or with other hydrophobic (natural or unnatural) amino acids, can be further augmented by use of the fusogenic peptides described *supra.*

### In vitro assembly of the vectors

One method for assembling the TAGD vector is via the use of micelle preparations containing the elements that are to be added to the vector. Thus, for example, the targeting ligand(s) and fusogenic element(s) can be covalently linked as described above to a linked polymer-lipid compound, and the resulting conjugated molecule then is used to prepare micelles. Those micelles are added to a preparation of the core moiety whereby the conjugate is inserted into the surface of the core moiety. Similar methods of "diffusive exchange" are described, for example in U.S. Patent No. 5,631,018.

Other methods to be employed in the process of assembling the TAGD are known in the art. For example, one method of modifying a naturally occurring viral envelope is by double detergent dialysis, as described in U.S. Patent No. 5,766,625. The lipids of the viral envelope may be modified by using a preparation of lipids and/or immunoprotective polymers and a detergent such as sodium cholate to partially solubilize the envelope. The detergent then is removed by exhaustive dialysis against phosphate-buffered saline (PBS), followed by insertion of fusogenic moieties and/or targeting moieties by partial micellation with sodium deoxycholate or other appropriate detergent. Detergent is then removed once again by exhaustive dialysis.

The term "partial micellation" refers to a viral membrane which is "softened" to allow incorporation of additional immunoprotective lipid or polymer components but that is not solubilized (micellized) to the point that the bilayer structure is lost. The process of partial micellation can be controlled by monitoring the scattering of light of the vesicles using a laser light scattering instrument. Sufficient detergent is introduced into the vesicle dispersion to maintain the light scattering signal. Loss of the light scatter signal indicates true solubilization and loss of bilayer structure. After partial micellation, the integrity of the TAGD particles in one embodiment can be verified by determining the viral titer.

Useful detergents are well known to those skilled in the art and include, but are not limited to, bile salts (sodium cholate, deoxycholate, taurocholate, etc.), CHAPSO, octylglucoside, TRTTON-X derivatives, etc. These detergents can be zwitterionic such as CHAPSO, or nonionic such as octylglucoside or Triton-X. Non-ionic detergents are preferred, as they are less likely to cause loss of integrity in the TAGD particle. The selection of the detergent is determined taking into account the compatibility of a particular detergent with the surface protein to be inserted.

One additional method of assembling the TAGD vector in one embodiment is to covalently link one or more of the immunoprotective, targeting and cell entry elements to viral proteins present in the surface of the viral particle. Thus, for example, the element to be added can be prepared so as to carry an activated carboxyl group, which then may be reacted with free amino groups on the side chains of lysine molecules in the viral protein. Other methods of linking molecules to proteins are well known in the art. For example, the side chains of surface lysine molecules can be reacted with 2-iminothiolane (Traut's reagent) to provide free sulfhydryl groups. Those groups can then be reacted with one or more of the immunoprotective, targeting and cell entry elements that carry maleimide groups.

Methods of preparing the TAGD vectors with the surface which contains novel functional elements include sonication or vortexing of an enveloped virus in the presence of addition lipids, whereby those additional lipids are introduced into the existing lipid membrane (see, for example, the methods described in Huang et al., Biochemistry. 8:344-52, (1969)). TAGD vectors that contain desired novel functional components may also be prepared by forming liposomes while in the presence of core containing particles. These methods include liposomes formation by detergent dialysis (see Kagawa et al. J. Biol. Chem. 246:5477-5487 (1971)); freeze/thaw methods (see Mayer et al. Biochimica et Biophysica Acta 817:193-6, (1985) and Bally et al in: Liposomes as drug carriers, edited by Gregoriadis, G.Chichester-NY-Brisbaine-Toronto-Singapore: John Wiley & Sons, pp. 841-854 (1988)); reverse phase evaporation (see Szoka et al. Biochimica et Biophysica Acta 601:559-571 (1980)). Finally, it has been shown that block-copolymers of leucine and glutamic acid can behave like lipids and form membranes. See Minoura, Langmuir, 14:2145-2147(1998). Accordingly, such block-copolymers can be used as membrane elements in the TAGD vector using the methods described above.

When the TAGD core is a retroviral vector, it may be produced in large quantity and purified by the methods described in U.S. Patent No. 5,661,022.

### Method of Administration of the Vectors

The TAGD vectors will be by methods that are well known in the art. Preferably, the vectors are administered parenterally, more preferably intravenously or intraarterially

### In vitro testing of the vectors

Methods of *in vitro* testing of the vectors of the invention are well known in the art. For example, the vector may contain a reporter gene or a selective marker which can be used to track successful insertion and expression of the vector in a target cell. For example, the vector can be engineered to contain a reporter gene such as β-plactosidase (β-gal), chloramphenicol acyl transferase (CAT), luciferase (luc), or green fluorescent protein (GFP). The vector is then applied to a population of target cells and the level of the reporter gene product expression is measured. For control purposes this expression may be compared with the expression obtained with a vector that lacks either or both of the targeting ligand and the cell entry element. one or all of the immunoprotective elements. Alternatively, the vector can contain a resistance marker, such as a neomycin resistance gene. Following application of the vector to the target cells, the cells are assayed for drug resistance.

### In vivo efficacy testing of the vectors

Methods of measuring the *in vitro* efficacy of the vectors of the invention are well known in the art. For example, when the vectors are used for the treatment of a disease in a mammal, efficacy of the vector can be determined by study of the amelioration of one or more symptoms of the disease. Advantageously, the *in vivo* efficacy can use measurement of defined clinical end points that are characteristic of the progress or extent of a disease. Candidate diseases for gene therapy are well known in the art. See, for example, Verma et al., Nature 389:239 (1997) and reference cited therein.

The present invention, thus generally described, will be understood more readily by reference to the following examples, which are provided by way of illustration and are not intended to be limiting of the present invention.

### Examples:

The experiments described below demonstrate that synthetic functional moieties can be inserted onto the surface of a core containing particles. Specifically, a retroviral vector was functionally modified by non-genetic means to contain on its surface the α-MSH peptidomimetic ligand. TAGD vectors targeted with this ligand are useful for the gene therapy of melanoma. In addition, the experiments describe the encapsulation of retroviral particles.

Three approaches were used to carry out these steps: (1) direct particle surface modification; (2) use of micelle intermediates for surface insertion; and (3) use of multifunctional and polyvalent linkers. These methods exploit methods previously used to prepare liposomal conjugates and chemical attachment of small peptides directly to the surface of living organisms. *See* Kashkin et al., Imnumologija N6, 37-40 (1987) and *Author's certificate USSR* N 145085. A-61 K39/39 (1988)).

### Conjugation of carrier molecules to targeting ligand

Conjugation of carrier molecules was achieved by reaction of maleimidoyl derivatives with thiol groups present on the targting ligand. PEG was conjugated to phosphatidylethanolamine using methods that are known in the art, and converted to a maleimido derivative (maleimidoyl-PEG-PE) using standard techniques. The resulting compound was further conjugated to Cys 598-616 peptide (ILNRLVQFVKDRISVVQAL) as a targeting ligand peptide. The conjugation was monitored using the property of the maleimidoyl group to absorb at 300 nm. (Hermanson, *supra).* Similar results were obtained for conjugation of lipid (i.e. DSPE)-PEG-SH with maleimidoylated analogs of α-MSH and other peptides, including α-MSH^{NLD}, a peptidomimetic analog of MSH ([Nle4, D-Phe7]-α-MSH, obtained from NovaBiochem, San Diego,CA).

The level of the conversion of the peptides in conjugates was calculated based on molar absorbance of maleimidoyl group (630 M/cm⁻¹). Completeness of the reaction is determined by measuring unbound thiol groups using Ellmann's reagent. Conjugates also were characterized by mass spectrometry (ES/MS and MALDI-TOF) to confirm that the conjugate contained no unbounded peptide that might interfere with the results of further experiments. Prepared conjugates were purified by gel-filtration in aprotic media and, in micellar form, by dialysis. *See* Karnoup et al., J. Peptide Res. 49:232-239 (1997).

The resulting lipid-PEG-α-MSH conjugate was shown to retain biological activity by assay for melanin formation in melanoma B16 cells. Thus, α-MSH, DSPE-PEG-SH, and DSPE-PEG-α-MSH conjugates were assayed at the same nanomolar concentrations. The α-MSH conjugate induced melanin formation with comparable efficiency to the non-conjugated α-MSH peptide.

### Incorporation of the conjugate into viral particles and cell targeting

The ability of thermodynamically unstable micelles to fuse with larger membrane surfaces was exploited for the next step in the construction of the TAGD vehicle. This method can be used to incorporate lipid-polymer-targeting ligand conjugates into the membrane surface of either liposomes *(see* Kirpotin, *supra)* or virions (see below).

The lipid-PEG-¹²⁵I α-MSH conjugate was incubated with MoMuLV viral particles. The modified virions were purified first by step sucrose gradient followed by discontinuous gradient centrifugation. The high isotope counts were shown to associate with the same fractions at which virions were pelleted.

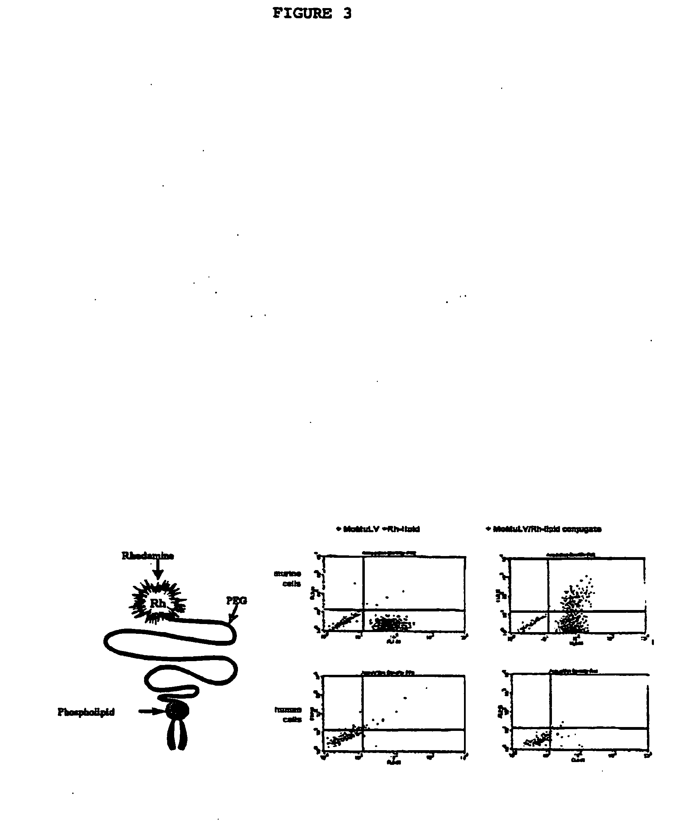

To further demonstrate the utility of this method, DSPE-PEG-rhodamine (red fluorescence) was associated with virions using the micelles. Thus, MoMuLV was incubated with DSPE-PEG-rhodamine and than allowed to bind to murine NIH 3T3 cells or human D10 melanoma cells. The cells were then incubated with anti-MoMuLV env antibody, followed by a FITC-labeled secondary antibody (green fluorescence). The resulting labeled cells (plus negative control cells) were analyzed by FACS. The results demonstrate (see Figure 3) that the rhodamine signal (red fluorescence in the upper right quadrant) was specifically detected on the murine cells containing viral receptors only when viral particles were allowed to incorporate the conjugate via incubation with micelles. Thus, DSPE-PEG-rhodamine enters the MoMuLV particles as expected, and can be detected on the murine host cells.

Next, a DSPE-PEG-α-MSH^{NLD} conjugate was incubated with MoMuLV viral particles and assayed to demonstrate the ability of the conjugate to redirect the murine retroviral particle to bind D10 human melanoma cells. The modified and non-modified MoMuLV particles were allowed to bind to the D10 cells and these cells were tested for the presence of the Moloney *env*-specific signal detected with anti-env primary nd FITC-labeled secondary antibodies (as described above). The FACS profiles demonstrated a positive shift on the human D10 cells following binding of the MoMuLV pre-treated with lipid-PEG-α-MSH conjugate. In a negative control, virions modified with lipid-PEG but lacking α-MSH did not exhibit similar binding to human cells. These experiments show that murine retroviral particles can be modified to bind human melanoma cells by surface incorporation of an α-MSH peptidomimetic conjugate. Accordingly, it was shown that it is possible to redirect surface-modified particles to new specific host cells.

The same particles were tested for the ability to transduce human cells. Conjugates of α-MSH linked through DSPE-PEG on the surface of recombinant viral particles (from the eco producing cell line GPE86/LNCX) provided viral titers > 10², indicating successful delivery of the marker genes (neo and β-gal). When a copolymer of glutamic acid with leucine was used as for presentation of α-MSH on the surface of MoMuLV titers > 10⁴of were obtained. The wild type MoMuLV titer of the non-modified and non-manipulated particle in murine NIH 3T3 cells is -10⁶. Details of the preparation of the copolymer-MSH conjugate are provided below.

An alternative approach to create TAGD vehicles uses chemical conjugation of functional peptides, peptidomimetics and polymers, to the surface of virions directly or through a novel polyfunctional and polyvalent linker. These chemical conjugation methods require the use of organic co-solvents and treatment with other reactive organic compounds and, thus, it first was necessary to establish working concentration of the chemical modifier reagents that sustain viral viability. Viruses were subjected to the treatment with a variety of organic solvents and conjugates. It was found that MoMuLV is surprisingly stable to treatment with organic solvents, retaining almost full infectivity after 30min treatment with up to 5 % (v/v) acetonitrile or DMF. These results indicate that the virus is able to withstand the conditions necessary to carry out chemical modifications of the virions.

It also was shown that MoMuLV infectivity is retained after modification with Traut's reagent. This shows that it is possible to covalently attach conjugates to virions via modification of the viral surface with Traut's reagent, followed by the reaction of the modified virus with maleimidoylated compounds. Optimal conditions for modification were found to be about 1-8 mM Traut's reagent.

Further experiments demonstrated that it was possible to incorporate an α-MSH peptidomimetic ligand onto the MoMuLV surface using a polyfunctional and polyvalent linker containing 90% glutamate (w/w) and 10% leucine. See, for example Bychkova et al., Mol. Biol. (Moscow) 14:278-286 (1980). Briefly, the polymer (100 mg) was dissolved in 2 ml DMF with 300 mg di-pentafluorophenylcarbonate. Diisopropylethylamine (DIPEA) was dissolved in 1 ml DMF and slowly added in 0.1 ml portions. After 40 min. the reaction product was precipitated with ether, washed with ether and pentane, and dried. The resulting activated linker (10 mg) was dissolved in 2 ml DMF. The peptide (α-MSH^{NLD}, 1 mg) was added, followed by 25 µl DIPEA. The reaction was shaken overnight at room temperature, and then stored at -20°C. Addition of the linker in this fashion ensured that some of the active pentafluorophenyl esters were still retained for further conjugations to the surface of the gene delivery vehicle.

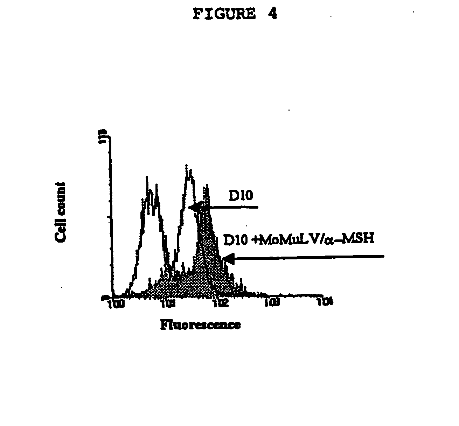

This linker-MSH conjugate then was incubated with the viral particle under similar conditions to those described above for DSPE-PEG-MSH. After modification, the MoMuLV particles were assayed for incorporation of α-MSH into the viral particle using Western blot analysis. The α-MSH positive signal was found to be associated with the *env* protein of the modified virus, but was not present on non-modified viruses to which non-conjugated α-MSH was added in a negative control. Addition of the MSH ligand in this fashion also successfully redirected the binding specificity of the modified MoMuLV particle. Thus, the chemically modified MoMuLV containing a polyfunctional and polyvalent linker containing the α-MSH peptidomimetic ligands was able to bind human melanoma D10 cells, whereas the unmodified virus did not. The binding was found to be superior using the polyfunctional linker compared to the chemical modifications described *supra.* These results are shown in Figure 4.

### Re-Encapsulation of MuLV Particles into a Novel Functional Surface

This experiment demonstrates that a gene-delivery particle such as a virion can be re-enveloped by a new functional surface. UV inactivated Sendai virus was used to provide such a functional retargeting and fusogenic enveloping surface to deliver the genetic content of MoMuLV particle to a novel host. Sendai virus previously has been demonstrated to fuse with bare membrane surfaces, and has been applied as a component of fusogenic liposomes in gene delivery (reviewed in Nakanishi et al.; Journal of Controlled Release 54:61-68 (1998)). UV-inactivated Sendai virus was used to fuse with MoMuLV encoding a green fluorescent protein (GFP) marker gene. When MoMuLV was added to a culture of Hela cells no GFP expression was detectable. However, fusion of MoMuLV with Sendai allowed GFP expression in human Hela cells (>50 cfu/ml), demonstrating transduction of a human cell line by the murine virus MoMuLV. Accordingly, these results demonstrate that re-enveloping of the core with artificial surfaces that contain fusion and retargeting molecules can be achieved.

## Claims

1. A non-naturally occurring gene therapy vector for cell-specific delivery of nucleic acid to a target cell,
comprising a recombinant viral core and a non-naturally occurring functional surface moiety,
wherein said core comprises a nucleic acid molecule,
wherein at least one expression product of said vector is a therapeutic nucleic acid, peptide or protein; and
wherein said functional surface moiety comprises
an immunoprotective element, which is a synthetic polymer moiety comprising a copolymer of glutamic acid with leucine,
a cell-entry element having a membrane destabilizing moiety comprising an amphiphilic α-helix and
a targeting element which is a peptide ligand for a cell surface receptor,
whereby the vehicle is capable of specifically binding to and delivering said core into a target cell.

2. The vector according to claim 1, wherein said viral core further comprises at least one viral capsid protein.

3. The vector according to claim 1, wherein polyethylene glycol is linked to said viral core by a linker comprising a copolymer of glutamic acid with leucine.

4. The vector according to claim 1, wherein said targeting peptide binds to a receptor that is more highly expressed in discussed cells than in normal cells.

5. The vector according to claim 1 wherein said targeting moiety is an RGD peptide.

6. The vector according to claim 1, wherein said amphiphilic α-helix is derived from the C-terminal domain of a viral env protein.

7. The vector according to claim 6, wherein C-terminal domain is the C-terminal domain of the Moloney leukemia virus env protein.

8. The vector according to claim 7, wherein said C-terminal domain comprises amino acids 598-616 of the Moloney leukemia virus env protein.

9. A vector according to claim 1 for the preparation of a pharmaceutical composition for treating a genetic disease, wherein said expression product is a product that is effective for treating said disease.

## Patentansprüche

1. Nicht in der Natur auftretender Gentherapie-Vektor für die zellspezifische Abgabe einer Nucleinsäure an eine Zielzelle,
umfassend einen rekombinanten viralen Kern und eine nicht in der Natur auftretende funktionelle Oberflächeneinheit,
wobei der Kern ein Nucleinsäuremolekül umfasst,
wobei mindestens ein Expressionsprodukt dieses Vektors eine therapeutische Nucleinsäure, Peptid oder Protein ist; und
wobei die funktionelle Oberflächeneinheit folgendes umfasst:
ein Immunoschutzelement, bei dem es sich um eine synthetische polymere Einheit handelt, die ein Copolymeres von Glutaminsäure mit Leucin umfasst,
ein Zellzugangselement mit einer Membran-destabilisierenden Einheit, die eine amphiphile α-Helix umfasst, und
ein abzielendes Element, bei dem es sich um einen Peptidliganden für einen Zelloberflächenrezeptor handelt,
wobei das Vehikel zur spezifischen Bindung an eine Zielzelle und zur Abgabe des Kerns an die Zielzelle befähigt ist.

2. Vektor nach Anspruch 1, wobei der virale Kern ferner mindestens ein virales Capsid-Protein umfasst.

3. Vektor nach Anspruch 1, wobei Polyethylenglycol mit dem viralen Kern über einen Linker, der ein Copolymeres von Glutaminsäure mit Leucin umfasst, verknüpft ist.

4. Vektor nach Anspruch 1, wobei das abzielende Peptid an einen Rezeptor bindet, der in den zur Diskussion stehenden Zellen stärker exprimiert wird als in normalen Zellen.

5. Vektor nach Anspruch 1, wobei es sich bei der abzielenden Einheit um ein RGD-Peptid handelt.

6. Vektor nach Anspruch 1, wobei sich die amphiphile α-Helix von der C-terminalen Domäne eines viralen env-Proteins ableitet.

7. Vektor nach Anspruch 6, wobei es sich bei der C-terminalen Domäne um die C-terminale Domäne des Moloney-Leukämie-Virus-env-Proteins handelt.

8. Vektor nach Anspruch 7, wobei die C-terminale Domäne die Aminosäuren 598-616 des Moloney-Leukämie-Virus-env-Proteins umfasst.

9. Vektor nach Anspruch 1 zur Herstellung einer pharmazeutischen Zusammensetzung zur Behandlung einer genetischen Krankheit, wobei es sich beim Expressionsprodukt um ein Produkt handelt, das bei der Behandlung dieser Krankheit wirksam ist.

## Revendications

1. Vecteur de thérapie génique non présent à l'état naturel pour un apport spécifique à une cellule d'un acide nucléique à une cellule cible,
comprenant un noyau viral recombinant et un fragment de surface fonctionnel non présent à l'état naturel,
dans lequel ledit noyau comprend une molécule d'acide nucléique,
dans lequel au moins un produit d'expression dudit vecteur est un acide nucléique thérapeutique, un peptide ou une protéine ; et
dans lequel ledit fragment de surface fonctionnel comprend
un élément immunoprotecteur, qui est un fragment de polymère synthétique comprenant un copolymère d'acide glutamique avec de la leucine,
un élément d'entrée de cellule ayant un fragment déstabilisateur de membrane comprenant une hélice α amphiphile et
un élément de ciblage qui est un ligand de peptide pour un récepteur de surface de cellule,
moyennant quoi le véhicule est capable de se lier spécifiquement audit noyau et d'apporter celui-ci dans une cellule cible.

2. Vecteur selon la revendication 1, dans lequel ledit noyau viral comprend au moins une protéine de capside virale.

3. Vecteur selon la revendication 1, dans lequel du poly(éthylène glycol) est lié audit noyau viral par un élément de liaison comprenant un copolymère d'acide glutamique avec de la leucine.

4. Vecteur selon la revendication 1, dans lequel ledit peptide de ciblage se lie à un récepteur qui est plus fortement exprimé dans les cellules évoquées que dans des cellules normales.

5. Vecteur selon la revendication 1, dans lequel ladite fraction de ciblage est un peptide RGD.

6. Vecteur selon la revendication 1, dans lequel ladite hélice α amphiphile est dérivée du domaine C-terminal d'une protéine env virale.

7. Vecteur selon la revendication 6, dans lequel le domaine C-terminal est le domaine C-terminal de la protéine env du virus de la leucémie de Moloney.

8. Vecteur selon la revendication 7, dans lequel ledit domaine C-terminal comprend les acides aminés 598 à 616 de la protéine env du virus de la leucémie de Moloney.

9. Vecteur selon la revendication 1 destiné à la préparation d'une composition pharmaceutique pour traiter une maladie génétique, dans lequel ledit produit d'expression est un produit qui est efficace pour traiter ladite maladie.
